# NEW EUROPEAN PATENT SPECIFICATION

(11) **EP 0 545 958 B2**
(45) Date of publication and mention of the opposition decision: **30.07.2003**
(45) Mention of the grant of the patent: 31.07.1996
(21) Application number: 91914361.0
(22) Date of filing: 27.08.1991
(51) Int. Cl.: C12N 9/42, D21C 9/10, C12S 3/08

(54) **A PROCESS FOR HYDROLYZING HEMICELLULOSE BY ENZYMES PRODUCED BY TRICHODERMA REESEI**
EIN PROZESS ZUR HEMIZELLULOSE MIT ENZYMEN, DIE DURCH TRICHODERMA REESEI PRODUZIERT WERDEN, ZU HYDROLYSIEREN
PROCEDE D'HYDROLYSE DE L'HEMICELLULOSE A L'AIDE D'ENZYMES PRODUITES PAR TRICHODERMA REESEI

(30) Priority: 27.08.1990 FI 904214
(43) Date of publication of application: 16.06.1993
(73) Proprietor: VALTION TEKNILLINEN TUTKIMUSKESKUS, 02151 Espoo (FI)
(72) Inventor: KANTELINEN, Anne, SF-02210 Espoo (FI); PERE, Jaakko, SF-01620 Vantaa (FI); POUTANEN, Kaisa, SF-00200 Helsinki (FI); TENKANEN, Maija, SF-02360 Espoo (FI); VIIKARI, Liisa, SF-00200 Helsinki (FI)
(74) Representative: Knuth-Lehtola, Sisko Hillevi
(86) International application number: FI9100265
(87) International publication number: WO92003541

(56) References cited:
- US-A- 4 797 361
- CHEMICAL ABSTRACTS, vol. 105, no. 23, 08 December 1986, Columbus, OH (US); A. LAPPALAINEN, p. 265, no. 205246t
- CHEMICAL ABSTRACTS, vol. 107, no. 15, 12 October 1987, Columbus, OH (US); K. POUTANEN et al., p. 581, no. 132597n
- CHEMICAL ABSTRACTS, vol. 109, no. 5, 01 August 1988, Columbus, OH (US); P. BIELY et al., p. 280, no. 34278f
- CHEMICAL ABSTRACTS, vol. 100, no. 11, 12 March 1984, Columbus, OH (US); H. ESTERBAUER et al., p. 424, no. 84137u
- CHEMICAL ABSTRACTS, vol. 111, no. 17, 23 October 1989, Columbus, OH (US); K. POUTANEN et al., p. 313, no. 149377w
- CHEMICAL ABSTRACTS, vol. 105, no. 1, 07 July 1986, Columbus, OH (US); M. HRMOVA et al., p. 317, no. 3269s
- Poutanen, K. (1988), Dissertation, Espoo 1988, Technical Research Centre of Finland, Publications 47
- Biely & Markovic, Biotechnol. Appl. Biochem.10 (1988) 107-117
- CHEMICAL ABSTRACTS, Volume 105, No. 23, 8 December 1986, (Columbus, Ohio, US), LAPPALAINEN, ARJA: "Purification and characterization of xylanolytic enzymes from Trichoderma reesei", see page 265, Abstract 205246t, & Biotechnol. Appl. Biochem. 1986, 8(5), 437-448.
- CHEMICAL ABSTRACTS, Volume 107, No. 15, 12 October 1987, (Columbus, Ohio, US), POUTANEN, KAISA et al.: "Evaluation of different microbial xylanolytic systems", see page 581, Abstract 132597n, & J. Biotechnol. 1987, 6(1), 49-60.
- CHEMICAL ABSTRACTS, Volume 109, No. 5, 1 August 1988, (Columbus, Ohio, US), BIELY, PETER et al.: "Resolution of glycanases of Trichoderma reesei with respect to cellulose and xylan degradation", see page 280, Abstract 34278f, & Biotechnol.Appl.Biochem. 1988, 10(2), 99-106.
- CHEMICAL ABSTRACTS, Volume 100, No. 11, 12 March 1984, (Columbus, Ohio, US), ESTERBAUER, HERMAN et al.: "Enzymic degradation of hemicelluloses by Trichoderma reesei enzymes", see page 424, Abstract 84137u, & Papier (Darmstadt) 1983, 37(12), 601-608.
- CHEMICAL ABSTRACTS, Volume 111, No. 17, 23 October 1989, (Columbus, Ohio, US), POUTANEN, KAISA et al.: "The xylanolytic enzyme system of Trichoderma reesei", see page 313, Abstract 149377w, & ACS Symp.Ser. 1989, 399(), 630-640.
- CHEMICAL ABSTRACTS, Volume 105, No. 1, 7 July 1986, (Columbus, Ohio, US), HRMOVA, MARIA et al.: "Specificity of cellulase and beta-xylanase induction in Trichoderma reesei", see page 317, Abstract 3269s, & Arch.Microbiol. 1986, 144(3), 307-311.

## Description

### FIELD OF THE INVENTION

The invention concerns a method for improving the bleachability of cellulose pulps and for improving the sheet properties of pulps using enzyme preparations which are composed essentially of one or two characterized xylanases produced by Trichoderma reesei.

### BACKGROUND OF THE INVENTION

Depending on the species, wood contains about 20% of hemicelluloses, of which xylans form an essential part both in hardwoods and softwoods. Hardwoods contain 15-30% glucuronoxylan and softwoods 7-10% arabinoglucuron-oxylan. During pulping processes, part of the hemicellulose is dissolved, but part remains within the fibres. The structure of the hemicelluloses remaining in the fibres affects their physical and functional properties. Depending on the type of pulping method used, soluble hemicellulose fractions differing with respect to their chemical composition and molecular weight distribution can be obtained. Even some dissolved hemicelluloses may have a high molecular weight, ie. these hemicelluloses are composed of polymeric sugar chains (xylans). Hemicellulose fractions can also be isolated from wood using methods especially developed for this purpose, e.g. for the production of xylose.

Depending on the composition of hemicellulose, several enzymes are needed for the hydrolysis. Of primary importance in many applications are the endo-β-xylanases (EC 3.2.1.8.), which hydrolyze the xylan backbone chains. Xylanases can be produced by several different micro-organisms. In an enzyme production process, these organisms usually produce several different hemicellulose-degrading enzymes into the culture broth. These contain enzymes hydrolyzing the backbone chain as well as enzymes capable of splitting off side chains or side groups attached to the backbone (endo-xylanases, β-xylosidase, arabinosidase, α-galactosidase, glucuronidase, acetyl xylan esterase etc). In addition, within a certain type of enzyme group, individual enzymes hydrolyzing the same substrate using a different mechanism can be isolated. Examples of such enzymes are e.g. the well documented cellulases (cellobiohydrolases I and II, endoglucanases I and II etc). These enzymes differ from each other with respect to both their biochemical structure and their mode of action.

Trichoderma reesei is frequently used for the production of both cellulases and hemicellulases. The ability of this organism to produce different enzymes has been widely described, as well as the methods for separation and purification of these enzymes, especially cellulases. By contrast, the biochemical properties of different xylanases produced by this organism and moreover, the exploitation of these specific properties for different modifications of wood-derived hemicelluloses is not well known.

Trichoderma reesei produces several xylan-degrading enzymes, of which, however, only two are specific for xylan (Biely and Markovic, 1988). The other known xylanases also degrade cellulose. which in pulp and paper applications often is a detrimental property. Different species of the fungus Trichoderma have been used for production of xylanases. Generally, one main xylanase has an isoelectric point (pl-value) above 8 (e.g. Gibson and McCleary, 1987, Dekker, 1985, Wong et al. 1986). In only one study has another xylanase, with an isoelectric point of 5.1, been purified and characterized (John and Smith, 1988), however, from a strain of Trichoderma lignorum.

In this invention, two functionally different xylanases and their applications are described. The invention is based on the unexpected observation that two xylanases produced by Trichoderma reesei possess essentially different properties which can be exploited in different practical applications.

In this invention the two xylanases were separated from each other using as such known protein purification methods. Anionic and cationic ion exchangers were used for separation of the proteins. The methods used were unexpectedly rapid and simple. This invention, however, is not limited to this protein purification method, but the desired proteins can also be purified by other methods.

The essential observation of this invention is the possibility to exploit the functional differences between these two xylanases in practical application processes. Xylanases can be characterized according to their optimal activities in different conditions or to their abilities to hydrolyze different substrates. In addition. enzymes may show different hydrolysis patterns for the same substrate. The enzymes described in this invention differ surprisingly from each other with respect both to their pH-optimae and to their xylan-solubilizing and saccharifying activities against different substrates. In this patent application, the enzymes are denominated following the international enzyme nomenclature. according to which the enzymes are numbered in the order of increasing isoelectric point (pl-value). Thus, the xylanase enzyme having the lower pl-value (pl 5.5) is named as xylanase I and the xylanase enzyme having the higher pl-value (pl 9.0) is named as xylanase II. It is characteristic of the enzymes described in this invention that one enzyme (xylanase I) has optimal activity in the more acid pH region, whereas the other (xylanase II) has its pH-optimum in the near-neutral pH range. Xylanase I typically solubilizes xylan more efficiently, whereas xylanase II produces reducing sugar units more efficiently. Xylanases I and II also differ from each other with respect to their ability to degrade chemically modified xylans with a low degree of substituents (side groups). Xylanase I is relatively more efficient in the hydrolysis of this type of modified substrate. These properties can advantageously be utilized in different applications, i.e. the most suitable enzyme can be chosen according to the conditions (pH) and the structural properties of the substrate to be treated with the enzymes. Depending on the process and the wood species used in the process, the chemical structure of the substrate (xylan) varies especially with respect to the amount and type of substituents on the xylan backbone. These structural differences are widely described in the literature of the field.

Several processes and methods based on the utilization of hemicellulases have been described in the literature. In these methods, the aim is to hydrolyze the hemicellulose to a greater or lesser extent. These methods include e.g. the partial hydrolysis of hemicelluloses from cellulose pulps to decrease the consumption of chlorine chemicals in bleaching or to decrease the chlorinated residues in the pulp or in the effluents (eg. Viikan et al. 1987, Clark et al. 1989, Tan et al. 1987), removal of residual hemicelluloses for the production of dissolving pulps (Paice and Jurasek. 1984. Paice et al. 1988), modification of fibre properties by a partial hydrolysis of hemicellulose (Noe et al. 1986, Fuentes and Robert, 1986, Mora et al. 1986, Pommier et al. 1989, Roberts et al. 1990) or hydrolyis of solubilized xylans to monomeric sugars. However, in the methods described, the enzymes used have consisted of undefined mixtures, and the identified specific properties of the individual enzymes have not been exploited. Neither have the enzymes been isolated from the Trichoderma reesei species. For example, in the method described by Tan et al. the cellulase-free xylanase preparation was prepared from a strain of Trichoderma harzianum. The xylanases produced were in no way characterized or separated from each other. The specific properties of individual xylanases have not been exploited in any previously published applications.

It is characteristic for the process described in this invention that, of the enzymes described here, either one or a mixture of the two may be used. The need for the enzymes depends essentially on the application being considered. A novel feature of the invented method is that of the two xylanases described the most favourable combination can be designed for each application. For example, when a high degree of hydrolysis of xylan is desired, according to the method invented, it is advantageous to prepare an enzyme mixture containing both the solubilizing activity and the saccharifying activity. When the substrate contains mainly soluble, small molecular weight oligosaccharides, it is advantageous to utilize mainly xylanase II. On the other hand, when only a partial hydrolysis of chemically modified, fibre-bound xylan is desired (as in the pretreatment for bleaching), it is advantageous to use only xylanase I or a mixture containing it. If the aim is to improve fibre properties by a partial hydrolysis of the xylan within the fibres (as also for reduced energy consumption in the production of mechanical pulps), it is more advantageous to use only xylanase II or a mixture containing it. As a basis for choosing the most advantageous enzyme, their different pH-optimae can also be exploited: xylanase I in the pH range of 3-6 and xylanase II in the pH range of 4-7, When a mixture of both is used. the pH can most advantageously be 3-7.

In particular, the invention concerns a method for improving the bleachability of cellulose pulps, wherein unbleached kraft pulp is treated with an enzyme preparation isolated from T. reesei and subjected to chemical, bleaching, and wherein the enzyme preparation contains xylanase I or xylanase II or a mixture of them.

The invention concerns also a method for improving the sheet properties of pulps wherein coarsly refined mechanical pulp is treated with an enzyme preparation isolated from T. reesei and subjected to refining and sheet preparation and wherein the enzyme preparation contains xylanase II or a mixture of xylanase I and xylanase II.

In the following the invention will be examined in more detail with the aid of non-limiting working examples. The isolation and characterization of the enzymes are described in examples 1, 2 and 3 and their applications in examples 4,5,6 and 7.

The xylanase activities of the enzymes are determined using two methods: the XYL-DNS method measuring the formation of reducing sugars from xylan, as described by Poutanen and Puls (1988), and the XYL-SOL method which measures the xylan solubilizing activity, as described by Bailey and Poutanen (1989). The saccharifying activity is the more common method used for determining xylanase activity both in the scientific literature and in the characterization of commercial xylanase preparations, although many variations of the method exist. Cellulase activity is analyzed as activity degrading hydroxyethylcellulose (IUPAC, 1987).

When comparing different enzymes, they can be dosed either on the basis of enzyme protein or activity units. The former method reflects the functional differences (specific activity), whereas the latter is more practical when comparing e.g. different commercial preparations.

### EXAMPLES

### Example 1. Purification of the enzymes.

Purification of the enzymes was started by chromatography using a cation exchanger (CM-Sepharose) at pH 4, adding sodium chloride to develop a linear concentration gradient between 0 and 0.15 M. The desired enzymes were collected into these fractions. The first xylanase (xylanase I) with a pl value later determined to be 5.5, was further chromatographically purified on an anion exchanger (DEAE.Sepharose) at pH 7.0. The second xylanase (xylanase II), pl value 9.0, was further purified on a cation exchanger (CM-Sepharose) at pH 8.0.

### Example 2. Characterization of the enzymes.

The protein properties of the enzymes purified according to example 1 were further characterized by standard methods used in protein chemistry. These properties are described in Table 1.

**Table 1.**

| Properties of the Trichoderma reesei xylanases. | | | |
|---|---|---|---|
| Enzyme | Isoelectric point, pl | Molecular weight (kDa) | pH-optimum |
| Xylanase I (Xyl I) | 5.5 | 19 | 4.0-4.5 |
| | | | |
| Xylanase II (Xyl II) | 9.0 | 20 | 5.0-5.5 |

### Example 3. Substrate specificities of the enzymes.

The properties of the enzymes purified and characterized acccording to examples 1 and 2 were further described according to their abilities to hydrolyze different substrates. The results are summarized in Table 2.

**Table 2.**

| Specific activities of the enzymes. | | | |
|---|---|---|---|
| Enzyme | Specific activity | | |
| | Cellulase HEC (nkat/mg) | Xylanase XYL/SOL (U/mg) | Xylanase XYL/DNS (nkat/mg) |
| Xylanase I | 0 | 630 | 1700 |
| Xylanase II | 0 | 430 | 7000 |

### Example. 4. (reference) Hydrolysis of isolated hemicellulose.

Xylan isolated from a waste liquor originating from hardwood was hydrolyzed using xylanases I and II isolated according to example 1. The amount of enzyme used was 150 µg/g of substrate dry weight when only one xylanase was used. When the two xylanases were applied, each of the enzymes was dosed at half of this amount. The hydrolysis experiments were carried out at 45 °C, at pH 5 and the hydrolysis time was 24 hours. The hydrolysis results, corresponding to the properties of the enzymes, are presented in Table 3. According to the results, it is obvious that when a high degree of hydrolysis is required, it is most advantageous to use a mixture of both enzymes. For a high degree of hydrolysis, both high solubilizing and saccharifying activities are needed in the enzyme preparation.

**Table 3.**

| Hydrolysis of waste liquor xylan. | |
|---|---|
| Enzyme | Hydrolysis result* (% of original substrate) |
| XYL I | 26 |
| XYL II | 33 |
| XYL I + XYL II | 40 |
| * In the hydrolysis result, the total amounts of small molecular weight hydrolysis products: xylose, xylobiose, xylotriose and xylotetraose were included. | |

### Example. 5. Bleaching of kraft pulp.

Unbleached pine sulphate pulp (kappa number 34.1) was treated at a consistency of 5 % with the T. reesei xylanases XYL I and XYL II at 45 °C for 4 hours. The original pH value was adjusted to the optimum of each enzyme. The enzymes were dosed at 2 or 10 µg protein/g dry pulp. The reducing sugars released in the enzymatic treatment are presented in Table 4. In this partial, incomplete hydrolysis of xylan, xylanase II was able to liberate somewhat more reducing sugars than xylanase I during the four hours' hydrolysis test, as expected when considering the better ability of xylanase II to liberate reducing sugars. Oligomeric compounds detected in the solubilized fraction from the pulp were formed slightly more after the treatment with the xylanase I.

In Table 4 the activity units, expressed both as solubilizing and saccharifying activity units, corresponding to the enzyme protein amounts used, are also presented. As the saccharifying activity is the most commonly used unit for describing the activity of an enzyme preparation, it is most convenient to use it also as a basis for comparison of individual enzymes.

**Table 4.**

| The amount of sugars liberated from unbleached pine kraft pulps after treatments with XYL I and XYL II 4 hours' treatment, enzyme dosage 2 µg/g and 10 µg/g pulp). | | | | |
|---|---|---|---|---|
| Enzyme | PROT. (µg/g) | XYL/SOL (U/g) | XYL/DNS (nkat/g) | Red. sugars (g/g pulp) |
| XYL I | 2 | 1.2 | 3.4 | 0.20 |
| XYL I | 10 | 6.3 | 17 | 0.40 |
| | | | | |
| XYL II | 2 | 0.9 | 14 | 0.24 |
| XYL II | 10 | 4.3 | 70 | 0.54 |

After the enzymatic treatments, the pulps were chemically bleached using a chlorine bleaching sequence, where in the prebleaching stage the amounts of chlorine gas and chlorine dioxide were the same (calculated as active chlorine). The entire bleaching sequence was: (D50/C50)EDED. In the reference bleaching the chlorination factor was 0.18. In the enzymatically treated pulp, the amount of active chlorine was decreased by about 20 %, resulting in a chlorination factor of 0.15. After bleaching, the brightness-values, viscosities and intermediate kappa numbers (describing the lignin content of pulp after the prebleaching stage) were determined. The bleaching results are presented in Table 5.

**Table 5.**

| Bleaching of pine kraft pulps using purified xylanases XYL I and XYL II in a bleaching sequence of (D50/C50) EDED. Original kappanumber 34.1. | | | | | |
|---|---|---|---|---|---|
| Enzyme/dosage | Chlorination factor | | Kappa after prebl. | Brightness (%) | Viscosity (dm³/kg) |
| XYL I | 2 µg/g | 0.15 | 5.3 | 90.4 | 1080 |
| | 10 µg/g | 0.15 | 5.1 | 90.8 | 1070 |
| | | | | | |
| XYL II | 2 µg/g | 0.15 | 5.3 | 90.4 | 1080 |
| | 10 µg/g | 0.15 | 5.0 | 90.8 | 1070 |
| | | | | | |
| Reference | | 0.18 | 4.7 | 90.0 | 1050 |
| Reference | | 0.15 | 6.6 | 89.0 | 1070 |

It is obvious from the results that both the xylanases, when dosed as protein were able to increase the bleachability of kraft pulp equally efficiently. When, however, the xylanases were dosed on the basis of XYL/DNS activity (according to Table 2) it can be concluded that xylanase I acts more efficiently in the bleaching. Using the same dosage of the saccharifying XYL/DNS activity, 14-17 nkat/g of substrate (equivalent to 10 µg of xylanase I and 2 µg of xylanase II), a better bleaching result can be obtained with xylanase I.

### Example 6. Bleaching of kraft pulp.

Unbleached kraft pulp was enzymatically treated at a consistency of 2.5 % according to example 5 with xylanases I and II at pH-values of 3-7, at 45 °C for two hours. The enzymes were dosed at 100 nkat/g (XYL/DNS). After the enzymatic treatments the pulps were bleached according to example 5 and the final brightness values were determined. The results are presented in Figure 1. It can be seen that xylanase I is more efficient in the acid pH region, whereas xylanase II acts better in the neutral pH range.

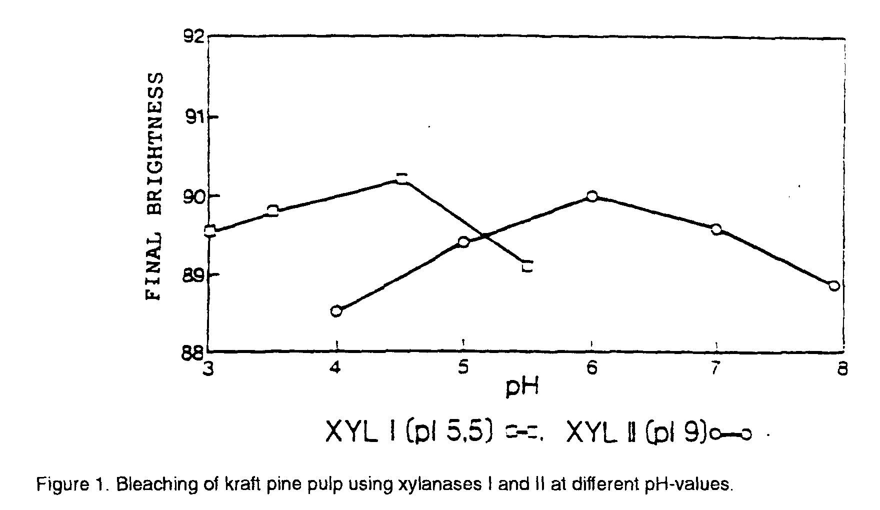
92
91
90
89
88
FINAL BRIGHTNESS
3 4 5 6 7 8
pH
XYL I (pI 5,5). XYL II (pI 9) .
Figure 1. Bleaching of kraft pine pulp using xylanases I and II at different pH-values.

### Example 7. Improvement of fibre properties of mechanical pulp.

Coarsly refined spruce mechanical pulp (TMP, freeness 450) was treated with purified xylanases I and I and II at their pH-optimae at 45°C for two hours. The enzyme dosage was 500 nkat/g of pulp (XYL/DNS). After the enzymatic treatments the pulps were refined in a PFI-refiner to a freeness value of about 100. The parameters describing the sheet properties were determined. The results are presented in Table 6. The percentage values in parentheses describe the extent of the positive (> 100%) or negative (< 100%) effect.

**Table 6.**

| The properties of mechanical pulps treated with xylanases. | | | |
|---|---|---|---|
| Treatment | Tensile index (Nm/g) | Tear index (mNm²/g) | Zero-span Tensile index (N/mg) |
| Reference | 25.6 | 5.04 | 81.3 |
| XYL I | 23.0 (90%) | 4.27 (85%) | 78.6 (97%) |
| XYL II | 26.5 (104%) | 5.24 (104%) | 85.5 (105%) |

As is obvious from the results, xylanase II had a clear positive effect on the paper technical properties of the TMP-pulp. The xylan in mechanical pulp resembles native xylan, with a negligible loss of side chains.

### REFERENCES

Bailey, M.J. & Poutanen, K., Appl. Microbiol. Biotechnol. 1989, 30: 5-10.
Biely, P. & Markovic, O., Biotechnol. Appl. Biochem. 1988, 10: 99-106.
Clark, T.A., McDonald, A.G., Senior, D.J., Mayers, P.R., Abstr. Fourth Int. Conference on Biotechnology in the Pulp and Paper Industry, Raleigh, 1989, pp. 39-40.
Dekker, R.F.H. 1985 Biodegradation of the hemicelluloses. In: Biosynthesis and biodegradation of Wood components. T. Higuchi (ed.), Academic Press, Inc. Orlando, pp. 505-533
Fuentes, J-L. & Robert, M. French Patent 8613208, 1986.
Gibson, T.S. & Mc Cleary, B.V., Carbohydr. Polymer. 1987, 7: 225-240.
IUPAC (International Union of Pure and Applied Chemistry). Pure Appl. Chem. 1987, 59: 257-268.
John, M. & Schmidt, J. (1988) Methods Enzymol. 160A: 662-671.
Mora, F., Comtat, J., Barnoud, F., Pla, F. & Noe, P. J. Wood Sci. Technol. 1986, 6, 147-165.
Noe, P., Chevalier, J., Mora, F. & Comtat, J. J. Wood Sci. Technol. 1986, 6: 167-184.
Paice, M.G., Bernier, R. & Jurasek, L. Biotechnol. Bioeng. 1988, 32: 235-239.
Paice, M.G. & Jurasek, L. J. Wood Sci. Technol. 1984, 4: 187-198.
Pommier, J.-C., Fuentes, J.-L. & Goma, G. Tappi J. 1989, June: 187-191.
Poutanen, K. & Puls, J., Appl. Microbiol. Biotechnol. 1988, 28: 425-432.
Roberts, J.C., McCarthy, A.J., Flynn, N.J. & Broda, P., Enz. Microb. Technol. 1990, 12: 210-213.
Tan, L.U.L., Wong, K.K.Y., Yu, E.K.C. & Saddler, J.N., Enzyme Microb. Technol. 1985, 7: 425-430.
Tan, L.U.L., Yu, E.K.C., Louis-Seize, G.W. & Saddler, J.N., Biotechnol. Bioeng. 1987, 30: 96-100.
Viikari, L., Ranua, M., Kantelinen, A., Linko, M. & Sundquist, J. Proc. 4th Int. Congr. on Wood and Pulping Chemistry, Paris, 1987. Vol. I, pp. 151-154.
Wong, K.K.Y., Tan, L.U.L. & Saddler, J.N., Can. J. Microbiol. 1986, 32: 570-576.

## Claims

1. A method for improving the bleachability of cellulose pulps, wherein unbleached kraft pulp is treated with an enzyme preparation isolated from *Trichoderma reesei* and subjected to chemical bleaching, and wherein the enzyme preparation contains an endo-β-xylanase I with a pI value of 5.5 and a molecular weight of 19 kDa.

2. The process according to claim 1, wherein the enzyme treatment is carried out in the pH range of 3 to 6.

3. The process according to any one of claims 1 or 2, wherein the enzyme treatment is carried out in the pH range of 4 to 5.

4. A process for improving the sheet properties of pulps, wherein coarsly refined mechanical pulp is treated with an enzyme preparation isolated from *Trichoderma reesei* and subjected to refining and sheet preparation, and wherein the enzyme preparation contains an endo-β-xylanase II with a pI value of 9.0 and a molecular weight of 20 kDa.

5. A method for improving the bleachability of cellulose pulps, wherein unbleached kraft pulp is treated with an enzyme preparation isolated from *Trichoderma reesei* and subjected to chemical bleaching, and wherein the enzyme preparation contains an endo-β-xylanase II with a pI value of 9.0 and a molecular weight of 20 kDa.

6. The process according to claim 4 or 5, wherein the enzyme treatment is carried out in the pH range 4-8.

7. The process according to any one of claims 4 to 6, wherein the enzyme treatment is carried out in the pH range of 5 to 7.

8. The process according to claim 4, 6 or 7, **characterized by** using a mixture of xylariase I and xylariase II.

9. The process according to claim 1,2 or 3 and 5, 6 or 7, **characterized by** using a mixture of xylanase I and xylanase II.

10. The process according to claims 8 or 9,**characterized by** using the mixture in the pH range of 3 to 8.

11. The process according to claims 8, 9, or 10, **characterized by** using the mixture in the pH range of 4 to 7.

## Patentansprüche

1. Verfahren zur Verbesserung der Bleichfähigkeit von Cellulosezellstoffen (Cellulosebrei, Pulpe), wobei der ungebleichte Kraftzellstoff mit einer aus *Trichoderma reesei* isolierten Enzymzubereitung behandelt und einem chemischen Bleichverfahren unterzogen wird, und wobei die Enzymzubereitung eine Endo-β-Xylanase I mit einem pl-Wert von 5,5 und einem Molekulargewicht von 19 kDa enthält.

2. Verfahren nach Anspruch 1, wobei die Enzymbehandlung bei einem pH-Wert im Bereich von 3 bis 6 durchgeführt wird.

3. Verfahren nach Anspruch 1 oder 2, wobei die Enzymbehandlung bei einem pH-Wert im Bereich von 4 bis 5 durchgeführt wird.

4. Verfahren zur Verbesserung der Blatteigenschaften von Zellstoffen, wobei der grob gemahlene mechanische Zellstoff (Holzstoff) mit einer aus *Trichoderma* reesei isolierten Enzymzubereitung behandelt und einer Refinermahlung und einer Blattherstellung unterzogen wird, und wobei die Enzymzubereitung eine Endo-β-Xylanase II mit einem pl-Wert von 9,0 und einem Molekulargewicht von 20 kDa enthält.

5. Verfahren zur Verbesserung der Bleichfähigkeit von Cellulosezellstoffen, wobei der ungebleichte Kraftzellstoff mit einer aus *Trichoderma reesei* isolierten Enzymzubereitung behandelt und einem chemischen Bleichverfahren unterzogen wird, und wobei die Enzymbehandlung eine Endo-β-Xylanase II mit einem pl-Wert von 9,0 und einem Molekulargewicht von 20 kDa enthält.

6. Verfahren nach Anspruch 4 oder 5, wobei die Enzymbehandlung bei einem pH-Wert im Bereich von 4 bis 8 durchgeführt wird.

7. Verfahren nach einem der Ansprüche 4 bis 6, wobei die Enzymbehandlung bei einem pH-Wert im Bereich von 5 bis 7 durchgeführt wird.

8. Verfahren nach Anspruch 4, 6 oder 7, **dadurch gekennzeichnet, dass** ein Gemisch aus Xylanase I und Xylanase II verwendet wird.

9. Verfahren nach den Ansprüchen 1, 2 oder 3 und 5, 6, 7 oder 8, **dadurch gekennzeichnet, dass** ein Gemisch aus Xylanase I und Xylanase II verwendet wird.

10. Verfahren nach Anspruch 8 oder 9, **dadurch gekennzeichnet, dass** das Gemisch bei einem pH-Wert im Bereich von 3 bis 8 verwendet wird.

11. Verfahren nach den Ansprüchen 8, 9 oder 10, **dadurch gekennzeichnet, dass** das Gemisch bei einem pH-Wert im Bereich von 4 bis 7 verwendet wird.

## Revendications

1. Procédé pour améliorer l'aptitude au blanchiment de pulpes de cellulose, dans lequel une pulpe kraft non blanchie est traitée avec une préparation enzymatique isolée à partir de *Trichoderma reesei*, et soumise à un blanchiment chimique, et dans lequel la préparation enzymatique contient une endo-β-xylanase I avec une valeur de pI de 5,5 et un poids moléculaire de 19 kDa.

2. Procédé selon la revendication 1, dans lequel le traitement enzymatique est effectué dans la gamme de pH de 3 à 6.

3. Procédé selon les revendications 1 ou 2, dans lequel le traitement enzymatique est effectué dans la gamme de pH de 4 à 5.

4. Procédé pour améliorer les propriétés de mise en feuilles de pulpes, dans lequel une pulpe mécanique grossièrement raffinée est traitée avec une préparation enzymatique isolée à partir de *Trichoderma reesei* et soumise à un raffinage et à une préparation de feuilles, et dans lequel la préparation enzymatique contient une endo-β-xylanase II avec une valeur de pI de 9,0 et un poids moléculaire de 20 kDa.

5. Procédé pour améliorer l'aptitude au blanchiment de pulpes de cellulose, dans lequel une pulpe kraft non blanchie est traitée avec une préparation enzymatique isolée à partir de *Trichoderma reesei* et soumise à un blanchiment chimique, et dans lequel la préparation enzymatique contient une endo-β-xylanase II avec une valeur de pI de 9,0 et un poids moléculaire de 20 kDa.

6. Procédé selon la revendication 4 ou la revendication 5, dans lequel le traitement enzymatique est effectué dans la gamme de pH de 4 à 8.

7. Procédé selon l'une quelconque des revendications 4 à 6, dans lequel le traitement enzymatique est effectué dans la gamme de pH de 5 à 7.

8. Procédé selon les revendications 4, 6 ou 7, **caractérisé par** l'utilisation d'un mélange de xylanase I et de xylanase II.

9. Procédé selon les revendications 1, 2 ou 3 et 5, 6 ou 7, **caractérisé par** l'utilisation d'un mélange de xylanase I et de xylanase II.

10. Procédé selon les revendications 8 ou 9, **caractérisé par** l'utilisation du mélange dans la gamme de pH de 3 à 8.

11. Procédé selon les revendications 8, 9 ou 10, **caractérisé par** l'utilisation du mélange dans la gamme de pH de 4 à 7.
